(19) Europäisches Patentamt
European Patent Office
Office européen des brevets

(11) EP 4 675 540 A1

(12) # EUROPEAN PATENT APPLICATION
published in accordance with Art. 153(4) EPC

(43) Date of publication:
**07.01.2026 Bulletin 2026/02**

(21) Application number: **23925406.3**

(22) Date of filing: **11.12.2023**

(51) International Patent Classification (IPC):
***G06Q 50/02*** *(2024.01)*

(52) Cooperative Patent Classification (CPC):
**G06Q 50/02**

(86) International application number:
**PCT/JP2023/044184**

(87) International publication number:
**WO 2024/180854 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2023 JP 2023029301**

(71) Applicant: **Seiwa Company, Limited**
**Shimotsuke-shi, Tochigi 329-0412 (JP)**

(72) Inventors:
- **NIIMURA, Subaru**
  **Shimotsuke-shi, Tochigi 329-0412 (JP)**
- **ODE, Hironobu**
  **Shimotsuke-shi, Tochigi 329-0412 (JP)**

(74) Representative: **Hoffmann Eitle**
**Patent- und Rechtsanwälte PartmbB**
**Arabellastraße 30**
**81925 München (DE)**

(54) ## CARBON DIOXIDE DYNAMICS SIMULATION DEVICE, COMPUTER PROGRAM, AND RECORDING MEDIUM

(57) Dynamics of carbon dioxide in a greenhouse are grasped. There are included: an absorption amount calculation unit 11 which finds an amount of net photosynthesis of a plant cultivated in the greenhouse as an absorption amount of carbon dioxide based on the plant; and a use rate calculation unit 12 which finds a rate of the absorption amount to a supply amount of carbon dioxide supplied into the greenhouse from a carbon dioxide supply source as a use rate of the carbon dioxide supplied to the greenhouse. The absorption amount and the use rate of carbon dioxide supplied to the greenhouse can be found, which makes it possible to clearly grasp the dynamics of carbon dioxide.

FIG. 1

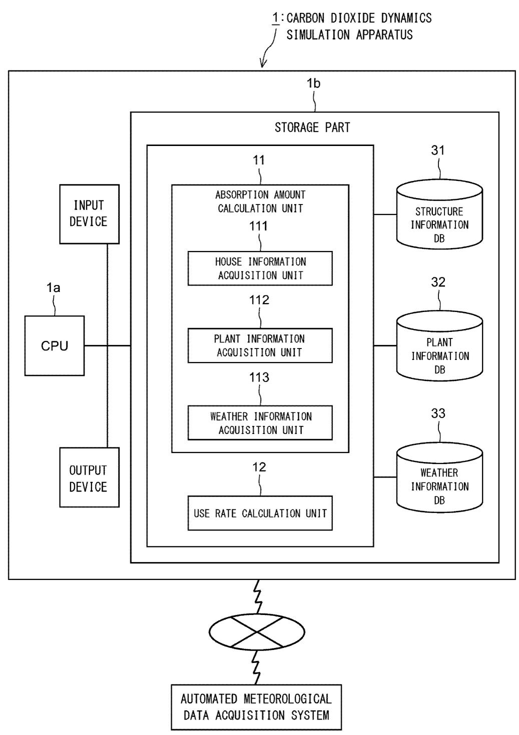


EP 4 675 540 A1

## Description

### Technical Field

The present invention relates to a technique of simulating dynamics of carbon dioxide in a greenhouse.

### Background Art

Various countermeasures against global warming are being studied as a pressing issue. In particular, a reduction in greenhouse effect gas in power stations, substations, factories, incineration plants, and the like which emit the greenhouse effect gas such as carbon dioxide can be expected to have a great effect on the countermeasures against global warming.

Patent Document 1 discloses a waste combined-incineration treatment system composed of an incineration treatment system equipped with an incinerator which subjects waste such as municipal waste, sewage sludge, and industrial waste to incineration treatment, and a methane fermentation treatment system equipped with a methane fermentation tank which subjects wet waste to methane fermentation treatment. Resource saving is attempted through reuse in the system, such as use of, by recovering and separating a methane gas and carbon dioxide, this separated carbon dioxide for cleaning ash produced in the incineration treatment system.

Patent Document 2 discloses a technique in which heat exhaust air containing a large amount of carbon dioxide of thermal power stations is not emitted to the air, and heat exchange to cause a drop to a predetermined temperature by using seawater is performed, and this heat exhaust air containing carbon dioxide is supplied to a plant factory to inhibit the emission to the air of the greenhouse effect gas.

Patent Document 3 discloses a technique of separating carbon dioxide from an exhaust gas emitted from thermal power stations, garbage incineration plants, and the like using a water film provided with a carbon dioxide absorption capacity, and also describes that the separated carbon dioxide is used for plant growth, thereby being used effectively without emitting carbon dioxide into the air to solve global environment problems.

Non-Patent Document 1 discloses, as one of global warming prevention techniques, a technique of supplying heat generated by combustion of gas or the like to a greenhouse to provide heating, and taking out carbon dioxide from an exhaust gas and supplying it to promote growth of plants.

### Prior Art Document

#### Patent Document

Patent Document 1: Japanese Patent Application Laid-open No. 2006-212524
Patent Document 2: Japanese Patent Application Laid-open No. 3-236723
Patent Document 3: Japanese Patent Application Laid-open No. 2021-133314

#### Non-Patent Document

Non-Patent Document 1: ene-fro, ENERGY FRONTLINE, April 13th, 2021, Vol. 25, "Tomato grows more and more rapidly with CO2! What is the latest technology of killing three birds with one stone?" URL: https://ene-fro.com/article/ef195_a1/

### Summary of the Invention

#### Problems to Be Solved by the Invention

The techniques disclosed in Patent Documents 1 to 3 and Non-Patent Document 1 are techniques of basically inhibiting an emission amount of a greenhouse effect gas into the air by recovering carbon dioxide and heat emitted from various energy emission facilities and using them for other uses. Among these, Patent Documents 2 to 3 and Non-Patent Document 1 are common to one another in that an emission amount to the air of carbon dioxide is inhibited by supplying carbon dioxide to plants.

Even use of a capacity for plants to absorb carbon dioxide also requires construction of a greenhouse capable of securing a suitable cultivation area if it aims to enhance an effect of inhibiting the emission amount of carbon dioxide to the air. However, the larger the cultivation area becomes, the more a construction cost of the greenhouse naturally increase, and for the construction, a greenhouse capable of securing a cultivation area with a size in which the carbon dioxide emitted from the energy emission facilities can be used effectively at a maximum is desired.

However, in the above-described conventional techniques, the concept of consuming carbon dioxide by cultivation of the plants is presented alone, and the study of how much carbon dioxide supplied to the greenhouse is absorbed depending on the plants is not made at all.

This is also the reason why a technique of preventing global warming by putting the greenhouse to practical use is not sufficient in terms of a rate of spread. In a case of aiming to construct the greenhouse for global warming prevention measures, making it possible to estimate beforehand how much a reduction in carbon dioxide can be achieved when a recoverable exhaust gas is used in the greenhouse causes a motivation for the construction of a new greenhouse. At this time, if an effect of the reduction in carbon dioxide can be grasped in terms of cost, the effect can also be expected to further increase. Naturally, also in the existing greenhouses, if a reduction amount of carbon dioxide and an economic effect in a case of using an exhaust gas from a neighboring energy emission facility can be grasped, the use of the exhaust gas is expected to be further promoted.

On the other hand, in the greenhouse, in order to promote photosynthesis, carbon dioxide is supplied by installing a carbon dioxide generator. The use of an exhaust gas of a heater is also already made. That is, even when an exhaust gas of other facilities such as factories is not used, carbon dioxide is actively supplied using a device operated by fossil fuel for the photosynthesis promotion. However, it is difficult to grasp how much the photosynthesis of plants is promoted by the supplied carbon dioxide numerically one by one, which is not sufficient in terms of practical application.

The present invention was made in consideration of the above, and has an object to provide a carbon dioxide dynamics simulation apparatus capable of grasping dynamics of carbon dioxide in a greenhouse, and promoting recycling use of carbon dioxide in an exhaust gas of power stations, factories, and the like to allow a contribution to a reduction in greenhouse effect gas, and to provide a computer program and a recording medium.

Means for Solving the Problems

To solve the above problems, a carbon dioxide dynamics simulation apparatus of the present invention is a carbon dioxide dynamics simulation apparatus which simulates dynamics of carbon dioxide in a greenhouse, the carbon dioxide dynamics simulation apparatus includes:

an absorption amount calculation unit which finds an amount of net photosynthesis of a plant cultivated in the greenhouse as an absorption amount of carbon dioxide based on the plant; and
a use rate calculation unit which finds a rate of the absorption amount to a supply amount of carbon dioxide supplied into the greenhouse from a carbon dioxide supply source during a predetermined period as a use rate of the carbon dioxide supplied to the greenhouse.

Preferably, the absorption amount calculation unit is configured to find the amount of net photosynthesis using a global solar irradiance amount, a light transmittance of the greenhouse, a light interception rate of a plant, and an indoor carbon dioxide concentration in the greenhouse.

Preferably, the indoor carbon dioxide concentration is calculated based on an indoor-outdoor ventilation rate of air in the greenhouse, and
the ventilation rate is calculated using a difference between an outdoor specific enthalpy and an indoor specific enthalpy of the greenhouse.

Preferably, the absorption amount calculation unit includes:

a house structure information acquisition unit which acquires structure information of the greenhouse including the light transmittance;
a plant information acquisition unit which acquires information of the plant targeted for cultivation including the light interception rate; and
a weather information acquisition unit which acquires weather information in an installation region of the greenhouse.

Preferably, the carbon dioxide dynamics simulation apparatus includes:

a first greenhouse-effect-gas emission-amount calculation unit which finds a first greenhouse effect gas emission amount in a case of providing a supply amount of carbon dioxide supplied into the greenhouse using a device which generates carbon dioxide by combustion of a fossil fuel as the carbon dioxide supply source;
a second greenhouse-effect-gas emission-amount calculation unit which finds a second greenhouse effect gas emission amount in a case of providing a supply amount of carbon dioxide supplied into the greenhouse with carbon dioxide recovered from an exhaust gas emitted from an exhaust gas generation source as the carbon dioxide supply source; and

a greenhouse-effect-gas reduction-amount calculation unit which calculates a difference between the first greenhouse effect gas emission amount and the second greenhouse effect gas emission amount as a greenhouse effect gas reduction amount in the case of using the exhaust gas.

Preferably, the first greenhouse-effect-gas emission-amount calculation unit finds a difference between a supply amount of the carbon dioxide and an absorption amount of the carbon dioxide as the first greenhouse effect gas emission amount.

Preferably, the second greenhouse-effect-gas emission-amount calculation unit finds the second greenhouse effect gas emission amount by setting a value corresponding to an absorption amount of the carbon dioxide as a negative number.

Preferably, the carbon dioxide dynamics simulation apparatus includes a cost calculation unit which finds a reduction cost of a carbon dioxide supply cost by comparing the case of supplying a supply amount of the carbon dioxide using the fossil fuel with the case of supplying a supply amount of the carbon dioxide using the exhaust gas.

Further, the present invention provides

a computer program which causes a computer to function as a carbon dioxide dynamics simulation apparatus in a greenhouse, the computer program causing the computer to execute

a procedure for finding an amount of net photosynthesis of a plant cultivated in the greenhouse as an absorption amount of carbon dioxide based on the plant, and

a procedure for finding a rate of the absorption amount to a supply amount of carbon dioxide supplied into the greenhouse from a carbon dioxide supply source during a predetermined period as a use rate of the carbon dioxide supplied to the greenhouse.

Preferably, in the procedure for finding an absorption amount of the carbon dioxide, the amount of net photosynthesis is found using a global solar irradiance amount, a light transmittance of the greenhouse, a light interception rate of a plant, and an indoor carbon dioxide concentration in the greenhouse.

Preferably, the indoor carbon dioxide concentration is calculated based on an indoor-outdoor ventilation rate of air in the greenhouse, and

the ventilation rate is calculated using a difference between an outdoor specific enthalpy and an indoor specific enthalpy of the greenhouse.

Preferably, the computer program causes the computer to execute,

in the procedure for finding an absorption amount of the carbon dioxide,

a procedure for acquiring structure information of the greenhouse including the light transmittance,

a procedure for acquiring information of the plant targeted for cultivation including the light interception rate, and

a procedure for acquiring weather information in an installation region of the greenhouse.

Preferably, the computer program causes the computer to further execute

a procedure for finding a first greenhouse effect gas emission amount in a case of providing a supply amount of carbon dioxide supplied into the greenhouse using a device which generates carbon dioxide by combustion of a fossil fuel as the carbon dioxide supply source,

a procedure for finding a second greenhouse effect gas emission amount in a case of providing a supply amount of carbon dioxide supplied into the greenhouse with carbon dioxide recovered from an exhaust gas emitted from an exhaust gas generation source as the carbon dioxide supply source, and

a procedure for calculating a difference between the first greenhouse effect gas emission amount and the second greenhouse effect gas emission amount as a greenhouse effect gas reduction amount in the case of using the exhaust gas.

Preferably, the computer program causes the computer to further execute a procedure for finding a reduction cost of a carbon dioxide supply cost by comparing the case of supplying a supply amount of the carbon dioxide using the fossil fuel with the case of supplying a supply amount of the carbon dioxide using the exhaust gas.

Further, the present invention provides a computer-readable recording medium in which the computer program is recorded. The recording medium in which the computer program is stored may be a non-transitory recording medium. The non-transitory recording medium is not particularly limited, and there are cited recording mediums such as a flexible disk, a hard disk, a CD-ROM, an MO (magneto-optical disk), a DVD-ROM, and a memory card, for example.

Effect of the Invention

According to the present invention, it is possible to measure the absorption amount of carbon dioxide based on the plant in the greenhouse. Therefore, the use rate of carbon dioxide supplied to the greenhouse can be found by the supply amount and the absorption amount of carbon dioxide during the predetermined period. This makes it possible to quantitatively grasp the dynamics such as emission outside the greenhouse of carbon dioxide accompanying supply, absorption, and ventilation of carbon dioxide in the greenhouse. Further, a contribution to a reduction in greenhouse effect gas can be made by using the exhaust gas as the carbon dioxide supply source and absorbing it into the plant, and a reduction effect can be grasped quantitatively and moreover grasped economically by finding the supply amount of the exhaust gas, the absorption amount of carbon dioxide based on the plant, and the use rate, resulting in allowing a contribution to promotion of an installation of the greenhouse for using the exhaust gas of factories and the like.

Brief Description of Drawings

[FIG. 1] FIG. 1 is a block diagram illustrating a schematic configuration of a carbon dioxide dynamics simulation apparatus according to one embodiment of the present invention.
[FIG. 2] FIG. 2 is a diagram illustrating one example of an input screen and an output screen displayed on a display.
[FIG. 3] FIG. 3 is a flowchart for explaining a calculation process of a daytime heat-release amount in an absorption amount calculation unit.
[FIG. 4] FIG. 4 is a flowchart for explaining a calculation process of an indoor carbon dioxide concentration in the absorption amount calculation unit.
[FIG. 5] FIG. 5 is a flowchart for explaining a calculation process of an amount of net photosynthesis and an absorption amount of carbon dioxide based on a plant in the absorption amount calculation unit.
[FIG. 6] FIG. 6 is a flowchart for explaining a calculation process of a supply amount of carbon dioxide.
[FIG. 7] FIG. 7 is a block diagram illustrating a schematic configuration of a carbon dioxide dynamics simulation apparatus according to the other embodiment of the present invention.

Modes for Carrying out the Invention

Hereinafter, based on an embodiment of the present invention illustrated in the drawings, description will be made in more detail. FIG. 1 is a diagram illustrating a schematic configuration of a carbon dioxide dynamics simulation apparatus 1 according to one embodiment of the present invention. As illustrated in this diagram, the carbon dioxide dynamics simulation apparatus 1 of this embodiment is constituted of a computer (the kind of computer is not limited, and includes a personal computer, a microcomputer, a portable information terminal, and the like) including a processor (CPU) 1a and a storage part (a case of being referred to as "storage part" in this description means including both of volatile and nonvolatile recording mediums such as main storage and storage, and neither of them is restrictive) 1b.

Specifically, in the carbon dioxide dynamics simulation apparatus 1 of this embodiment, a computer program which executes procedures to cause the computer which is the carbon dioxide dynamics simulation apparatus 1 to function as an absorption amount calculation unit 11 and a use rate calculation unit 12 is stored in the storage part 1b. The computer program is normally stored in the nonvolatile recording medium such as a hard disk or an SSD which is built in or externally attached to the computer (carbon dioxide dynamics simulation apparatus 1), and is read and executed by the above-described processor 1a. Further, a storage location of various kinds of data may be a storage part connected via a communication line other than the storage part built in or externally attached to the carbon dioxide dynamics simulation apparatus 1.

The absorption amount calculation unit 11 finds an amount of net photosynthesis of a plant cultivated in a greenhouse as an absorption amount of carbon dioxide ($CO_2$) based on the plant. An amount of net photosynthesis P is an absorption amount of carbon dioxide actually absorbed by the plant, and can be found based on a global solar irradiance amount ($MJ/m^2$) (Io), a light transmittance ($\tau$) of a covering material of the greenhouse, a light interception rate ($\phi$) of a plant, an indoor carbon dioxide concentration (indoor $CO_2$ concentration: $C_{in}$) of the greenhouse, a coefficient of transformation ($m^3/J$) ($\mu$) to a photosynthetic rate, a factor related to a reverse reaction ($\delta$). A global solar irradiance amount ($MJ/m^2$) (Io) $\times$ light transmittance ($\tau$) of the covering material of the greenhouse is an incident solar heat quantity ($MJ/m^2$) of the greenhouse.

Specifically, the amount of net photosynthesis can be found using the following simultaneous equations (1) to (4).

(1) Merit and demerit of carbon dioxide (ventilation change amount of indoor-outdoor carbon dioxide (E))

$$E = V(C_{in} - C_{out})$$

(V: ventilation amount of air, $C_{out}$: carbon dioxide concentration in the air (a constant is available))
(2) An absorption amount of carbon dioxide (P)

$$P = Io \cdot \tau \cdot \phi \cdot \mu \cdot C_{in}/(C_{out} + \delta)$$

(3) A supply amount of carbon dioxide (S)

$$S = P + E$$

(4) A heat balance equation

$$Io \cdot \tau = k_{cover} \cdot 1/\beta \cdot (T_{in} - T_{out}) + V \cdot (H_{in} - H_{out}) \cdot \rho$$

($k_{cover}$: overall heat transfer coefficient of covering material, $\beta$: heat retention ratio (ratio of a floor area to a coverage area), $T_{in}$: indoor temperature, $T_{out}$: outdoor temperature, $H_{in}$: indoor specific enthalpy, $H_{out}$: outdoor specific enthalpy, $\rho$: air density)

The absorption amount calculation unit 11 includes a house information acquisition unit 111, a plant information acquisition unit 112, and a weather information acquisition unit 113.

The house information acquisition unit 111 acquires structure information of the greenhouse to be simulated. The structure information is the kind of roof material, the light transmittance and the average heat-release coefficient linked for each kind of roof material, the design floor area, the design coverage area, and the like, of the greenhouse. In this embodiment, sizes of the greenhouse such as the design floor area and the design coverage area may be regular sizes for simulation, or may be set so that sizes which can be roughly estimated from an area of a site planned for construction can be specified. Further, on the premise that exhaust heat from factories in a region planned for construction, and the like is used, the design floor area and the design coverage area of the greenhouse with a size in which heat quantity for heating required to cultivate a predetermined plant during a predetermined period can be provided by the exhaust heat can also be simulated, and the data can also be used. The structure information is stored in a structure information database 31, and the house information acquisition unit 111 gains access to the structure information database 31, and acquires the required information.

The plant information acquisition unit 112 acquires information of a plant targeted for cultivation. The information of the plant includes the light interception rate, a coefficient of transformation ($m^3/J$) to an amount of photosynthesis, and a ventilation temperature during the day (daytime ventilation temperature) suitable for cultivation of the plant, which are specified for each kind of plant, and these pieces of the information are stored in the plant information database 32 for each kind of plant. Note that the "ventilation temperature" is a daytime control temperature proper for the plant targeted for cultivation, and is used in finding an estimated value of a later-described daytime indoor air temperature.

The weather information acquisition unit 113 acquires weather information of a region where the greenhouse is installed. In the weather information, these pieces of the information including a daily daytime average air temperature, a daily maximum air temperature, a daily minimum air temperature, a daily total global solar irradiance amount, and a daily day length in the region are stored in the weather information database 33 to match the region. The weather information database 33 can be formed by capturing the required information from Automated Meteorological Data Acquisition System (AMeDAS) or the like of the region. Note that in this embodiment, the weather information acquisition unit 113 reads these pieces of the information by access to the weather information database 33, and it is also possible to use a scheme to read it by direct access to Automated Meteorological Data Acquisition System or the like of the region.

Note that a place name, the kind of plant, the kind of roof material of the greenhouse, the supply amount of carbon dioxide ($CO_2$), and the like are input using an appropriate input device, and displayed on an input screen of a display as illustrated in FIG. 2 (the left half of a screen in FIG. 2).

The use rate calculation unit 12 finds a rate of the absorption amount of carbon dioxide found by the above-described absorption amount calculation unit 11 to the supply amount of carbon dioxide supplied into the greenhouse from a carbon dioxide supply source during a predetermined period as a use rate. The supply amount of carbon dioxide is set by inputting an estimated supply amount from the carbon dioxide supply source on the input screen as described above. The absorption amount, the supply amount, and the use rate of carbon dioxide are output to an output screen corresponding to the right half of the screen in FIG. 2.

The carbon dioxide supply source corresponds to an exhaust gas generation source such as a power station, a substation, a factory, or an incineration plant when the exhaust gas is used, in addition to a device which generates carbon dioxide by combustion of a fossil fuel, such as a carbon dioxide generator.

Next, a simulation example of carbon dioxide dynamics using the carbon dioxide dynamics simulation apparatus 1 of this embodiment will be described.

[Calculation of carbon dioxide absorption amount]

The carbon dioxide absorption amount is the amount of net photosynthesis in this embodiment, and can be found using the above formulas (1) to (4). An example of calculation steps of the carbon dioxide absorption amount using the above formulas (1) to (4) will be described in the following.

(Acquisition of information)

A simulation executor or the like inputs necessary items on the input screen illustrated in FIG. 2 from the input device (S301 in FIG. 3). Input items are the place name (for example, Sapporo), the kind of plant (for example, tomato), and the kind of roof material (for example, an FRA). When these pieces of the information are input, the house information acquisition unit 111 gains access to the structure information database 31 (S302), reads the average heat-release coefficient (for example, 5) corresponding to the kind of roof material, and acquires the design floor area (for example, 1000 $m^2$) and the design coverage area (for example, 1890.58 $m^2$) of the greenhouse in a predetermined size for simulation (S303). Note that the "design floor area" is a design value of a floor area of the greenhouse planned for construction, and is a value calculated assuming it to be in, for example, a square floor shape in order to reduce a computation load. The "design coverage area" is a design value of an area, covered with a covering material, adding a side face, an end face, and, a ceiling face and a gable face forming a roof of the greenhouse together. The "average heat-release coefficients" are values each obtained by finding an overnight heating load coefficient obtained by dividing an overnight heating heat quantity by an overnight nighttime degree hour corresponding thereto and a coverage area, regarding a plurality of kinds of films (the roof material, a lining covering material (curtain)) within an experimental period, to average them for each kind of films (the roof material, the lining covering material (curtain)). In this embodiment, there was adopted a value found by an experiment from November 9th, 1980 to February 3rd, 1981 conducted by providing four cultivation beds in each of three A to C houses constructed in Oyama Factory Farm Field of SEIWA CO., LTD. (a surface area: 262 $m^2$, a floor area: 120 $m^2$, an outer coating film: an agricultural vinyl chloride film, a heating system: a warm-air heater, a shape of curtain: A house ... a single layer of agricultural vinyl film, B, C houses ... double shaft and double layer, a curtain position (lower layer): A, B houses ... 1.8 m (from the ground), C house ... 1.85 m (from the ground), an interlayer distance in a case of double layer: 20 cm). In the experiment, only a portion on each cultivation bed was covered with a black mulch, and turnip greens, lettuce, field peas, and kidney beans were each planted for each cultivation bed, and an average value of the heating load coefficient in each house was found to correspond to the kind of films, and when the heating load coefficient of the A house was set to 100%, it was found what percent value was obtained according to combination of various films as the heating load coefficient of each of the B, C houses, and this value was set as the "average heat-release coefficient".

The plant information acquisition unit 112 gains access to the plant information database 32 (S304), and acquires the daytime ventilation temperature (23°C) corresponding to "tomato" which is the input plant targeted for cultivation (S305).

The weather information acquisition unit 113 gains access to the weather information database 33 (or from the public weather data or the like) (S306), and acquires the daily daytime average air temperature (average outside air temperature found geometrically for each day using the daily maximum air temperature and the daily minimum air temperature obtained from weather data in the past year) and a daily day length (s) = 32400 s (a fixed value is adopted in this embodiment, and a value in consideration of latitude and seasons can also be used) of "Sapporo" (S307).

When these pieces of the information are acquired, the absorption amount calculation unit 11 further executes the following steps.

(Calculation of daytime heat-release amount)

First, a daily daytime indoor air temperature is found using the daily daytime average air temperature and the daytime ventilation temperature (23°C) (S308). The daily daytime indoor air temperature can be found by a machine learning model which is an approximate expression found by regression analysis in which the daily daytime indoor air temperature is set as an objective variable and the daily daytime average air temperature and the daytime ventilation temperature (23°C) are set as explanatory variables. According to the approximate expression found in this embodiment, when the daily daytime average air temperature is less than 5°C, it is found by (daily ventilation temperature - 2), and when the daily daytime average air temperature is 5°C or more, it is found by (-0.8224 × daily daytime average air temperature + 1.299 × daytime ventilation temperature + 0.03229 × square of daily daytime average air temperature - 18.94/daily daytime average air temperature). A calculation is made as "21°C" using the former expression because this example is an example in which the daily daytime average air temperature is -0.7291°C (S308).

Next, the heat retention ratio is found (S309). The heat retention ratio is a design floor area/design coverage area, and is found as 1000/1890.58 = 0.5289 in the above-described example.

Next, a daily daytime heat-release amount ($MJ/m^2$) is found by the following formula (S310).

The daily daytime heat-release amount ($MJ/m^2$) = (daily daytime indoor air temperature (°C) - daily daytime average air temperature (°C))/heat retention ratio × average heat-release coefficient of roof material × daily day length (s)/ 3600 × 4.186/1000 = 7.7384 ($MJ/m^2$)

(Calculation of daily solar heat balance)

Next, a daily incident solar heat ($MJ/m^2$) is obtained by multiplying the daily total global solar irradiance amount ($MJ/m^2$) of "Sapporo" acquired from the weather information database 33, the public weather data, or the like (found from weather data for a year) and a light transmittance of the roof material acquired from the structure information database 31 together (S401 in FIG. 4). A daily solar heat balance ($MJ/m^2$) is found as follows using this daily incident solar heat ($MJ/m^2$) and the daily daytime heat-release amount ($MJ/m^2$) found by S310 in FIG. 3 (S402).

In a case of daily daytime heat-release amount ($MJ/m^2$) > daily incident solar heat ($MJ/m^2$), a calculation is made as "0".

In a case of daily daytime heat-release amount ($MJ/m^2$) ≤ daily incident solar heat ($MJ/m^2$), it is found by "daily daytime heat-release amount ($MJ/m^2$) - daily incident solar heat ($MJ/m^2$)".

(Calculation of outdoor specific enthalpy)

Next, a daily outdoor specific enthalpy (J/kg) is found using the daily maximum air temperature and the daily minimum air temperature in the region acquired from the weather information database 33, the public weather data, or the like (S403). The outdoor specific enthalpy is found using the following learned model.

First, using a daytime average air temperature and a daytime absolute humidity acquired from each of meteorological offices at a plurality of observation points, a daytime specific enthalpy is found by a general formula:

$$H = 1.006 \cdot Td + (1.86) \cdot Td + 2501) \cdot x$$

(Td is the air temperature, x is the absolute humidity).

The obtained specific enthalpy is set as an objective variable, and a maximum air temperature and a minimum air temperature are acquired for each day, and both are set as explanatory variables. This operation is performed one by one by obtaining their respective pieces of data for about two years from the meteorological offices at the above-described plurality of observation points, to generate the learned model by linear regression. As one example, the learned model used in this embodiment is indicated as follows.

The outdoor specific enthalpy (J/kg): $H = 4211 + 1389 \cdot T_h + 382.4 \cdot T_l - 16.26 \cdot T_h^2 + 55.90T_1^2$ ($T_h$: daily maximum air temperature, $T_1$: daily minimum air temperature) (coefficient of determination = 0.9683)

The outdoor specific enthalpy is found using the daily maximum air temperature and the daily minimum air temperature in the region in this manner.

(Calculation of indoor specific enthalpy)

The indoor specific enthalpy (J/kg) is found using the daily daytime indoor air temperature found by S308 in FIG. 3 (S404). The indoor air temperature for finding the indoor specific enthalpy is found by generating the learned model by the linear regression using data of an actual greenhouse. Specifically, in a greenhouse operated by TOMATOPARK Co, Ltd. which is a company affiliated with the present applicant, the daytime indoor air temperature (objective variable), a day average outside air temperature (explanatory variable), and the ventilation temperature (explanatory variable) were measured for several tens of weeks, and the learned model was generated by the linear regression. Moreover, a relative humidity for finding x was set as a fixed value, and the indoor air temperature (Td) was divided into certain ranges to be regression-analyzed, resulting in generating the learned model in which the indoor specific enthalpy was set as an objective variable. As one example, the learned model used in this embodiment is indicated as follows.

The indoor specific enthalpy (J/kg): $H = 63.242Td^2 + 181.18Td + 16504$ (indoor air temperature Td = coefficient of determination in a range of 10 to 40°C = 0.9996)

(Calculation of ventilation rate)

Next, a daily ventilation rate ($m^3/m^2$) of indoor-outdoor air of the greenhouse is found (S405). The daily ventilation rate is found using a gap ventilation rate per hour ($m^3/hm^2$) specified to correspond to the kind of roof material acquired from the structure information database 31 by the house information acquisition unit 111 (for example: 1.8 $m^3/hm^2$) and the above-described daily day length (s) linked to "Sapporo" (32400 s) in addition to the daily solar heat balance ($MJ/m^2$), the outdoor specific enthalpy (J/kg), and the indoor specific enthalpy (J/kg) which have been found based on the above (S406, S407).

Specifically, first,
A comparison is made between

a value divided by a difference between the outdoor specific enthalpy (J/kg) and the indoor specific enthalpy (J/kg) (EA) = 1000000 × daily solar heat balance ($MJ/m^2$)/(outdoor specific enthalpy (J/kg) - indoor specific enthalpy (J/kg))/1.2 (density of air $kg/m^3$)
and
a value obtained by multiplying the gap ventilation rate ($m^3/hm^2$) and the daily day length (s) together (EB) = the daily ventilation rate ($m^3/m^2$) = gap ventilation rate ($m^3/hm^2$) × daily day length (s)/3600.

As a result of the comparison, in a case of EA < EB, the daily ventilation rate ($m^3/m^2$) = gap ventilation rate ($m^3/hm^2$) × daily day length (s)/3600 is adopted.

In a case of EA ≥ EB, the 1000000 × daily solar heat balance ($MJ/m^2$)/(outdoor specific enthalpy (J/kg) - indoor specific enthalpy (J/kg))/1.2 (density of air $kg/m^3$) is adopted.

(Calculation of indoor carbon dioxide concentration)

The absorption amount calculation unit 11 further finds a carbon dioxide supply amount per unit area from an input value of the carbon dioxide supply amount and the design floor area (S408), and by multiplying this value by the daily day length (s) = 32400 s, finds a daily carbon dioxide supply amount per unit area (S409). Note that when the carbon dioxide supply amount is in a unit of kg, it is found in terms of a unit of $m^3$ by being divided by a mass of carbon dioxide of 1 $m^3$ at 25°C at 1 atmospheric pressure (S410). Further, the weather information acquisition unit 113 acquires an outdoor carbon dioxide concentration of "Sapporo" (410 ppm) from the weather information database 33 (S411, S412).

The plant information acquisition unit 112 of the absorption amount calculation unit 11 gains access to the plant information database 32 to acquire the factor related to the reverse reaction (δ), the light interception rate (ϕ), and the coefficient of transformation, regarding "tomato" (S413 to S415). A light interception amount (MJ) is converted to the amount of photosynthesis ($m^3$) by the coefficient of transformation.

When the above-described pieces of information are obtained, the absorption amount calculation unit 11 calculates a daily indoor carbon dioxide concentration according to the following formula using them (S417).

The indoor carbon dioxide concentration = (-(light interception rate × daily incident solar heat × coefficient of transformation + daily ventilation rate × δ - daily carbon dioxide supply amount (per area) ($m^3/m^2$) - daily ventilation rate × indoor carbon dioxide concentration) + (square root of ((light interception rate × daily incident solar heat × coefficient of transformation + daily ventilation rate × δ - daily carbon dioxide supply amount (per area) ($m^3/m^2$) - daily ventilation rate × outdoor carbon dioxide concentration)$^2$ - 4 × daily ventilation rate × (-δ × daily carbon dioxide supply amount (per area) ($m^3/m^2$) - δ × daily ventilation rate × outdoor carbon dioxide concentration))/2/daily ventilation rate ($m^3/m^2$)

FIG. 4 illustrates one example of a calculation result obtained by applying numerical values concretely until finding the indoor carbon dioxide concentration with the absorption amount calculation unit 11. Thus, for example, an indoor carbon dioxide concentration = 0.004432509 is found.

(Calculation of daily amount of net photosynthesis)

Access is gained to the plant information database 32 by the plant information acquisition unit 112 to acquire the factor related to the reverse reaction (δ), the light interception rate (ϕ), and the coefficient of transformation, regarding "tomato" (S501 to S503 in FIG. 5). A light interception amount (MJ) is converted to the amount of photosynthesis ($m^3$) by the coefficient of transformation (S504).

Access is gained to the structure information database 31 by the house information acquisition unit 111 to acquire the design floor area (S505).

A daily amount of net photosynthesis is found using the daily incident solar heat ($MJ/m^2$) (S401) and the daily indoor carbon dioxide concentration (S417) to these pieces of data (S506).

In this embodiment, it is found by the following formula.

The daily amount of net photosynthesis ($m^3$) = daily incident solar heat ($MJ/m^2$) × light interception rate × coefficient of transformation ($m^3/J$) × daily indoor carbon dioxide concentration × design floor area/(δ + daily indoor carbon dioxide concentration)

(Calculation of carbon dioxide absorption amount)

The obtained daily amount of net photosynthesis ($m^3$) is a daily carbon dioxide absorption amount, and is multiplied by a conversion factor to be converted to a unit of kg (S507), and thereby the daily carbon dioxide absorption amount is found in a unit of kg (S508).

By adding daily carbon dioxide absorption amounts during the cultivation period (for example, a cultivation start date September 1st, a cultivation end date: next June 1st) together, a period carbon dioxide absorption amount during the overall cultivation period is found. As a simulation, the period carbon dioxide absorption amount is found in a unit of month (S509), and an annual carbon dioxide absorption amount can be found by integrating the ones for a year (S510). By calculating the carbon dioxide absorption amount in a certain period unit, for example, an actual cultivation period, a month, or a year in this manner, the use rate, a reduction amount, and the like of carbon dioxide can be compared in later-described use rate calculation unit 12, first greenhouse-effect-gas emission-amount calculation unit 13, second greenhouse-effect-gas emission-amount calculation unit 14, greenhouse-effect-gas reduction-amount calculation unit 15, and cost calculation unit 16.

FIG. 5 illustrates one example of a calculation result of a process of calculating the carbon dioxide absorption amount with the absorption amount calculation unit 11. In this example, the daily amount of net photosynthesis = 9.877358432 $m^3$ = 17.76936782 kg, the daily carbon dioxide absorption amount = 17.76936782 kg, the period carbon dioxide absorption amount for a month (an example of integration from January 1st to January 31st during the above-described cultivation period) = 0.65249752 t, and the annual period carbon dioxide absorption amount = 10.10189154 t are found. The absorption amount calculation unit 11 outputs and displays "10.1 t" in a section of "$CO_2$ absorption amount" of the output screen illustrated in FIG. 2 (S511).

[Calculation of use rate]

The use rate calculation unit 12 calculates a use rate using the carbon dioxide absorption amount obtained by the absorption amount calculation unit 11.

The use rate is found by a comparison with the carbon dioxide supply amount. The carbon dioxide supply amount is found as follows.

First, it is assumed that 15 kg/h is input as the carbon dioxide supply amount, for example (refer to the input screen in FIG. 2). This is divided by 1000 $m^2$ of the above-described design floor area to calculate an hourly supply amount per unit area of 0.015 $kg/hm^2$ (S601 in FIG. 6). Next, the day length of "Sapporo" (32400 s) is read from the weather information database 33 (S602) to find a daily hourly supply amount per unit area (S603), and moreover, by being multiplied by 1000 $m^2$ of the design floor area, a daily carbon dioxide supply amount of 135 kg is found (S604).

The daily carbon dioxide supply amount is found, and then the daily carbon dioxide supply amounts are integrated for an appropriate period, and similarly to the above-described carbon dioxide absorption amount, a period carbon dioxide supply amount is calculated in a unit of an actual cultivation period, a month, a year, or the like (S605 to S607). Thus, for example, a period carbon dioxide supply amount for a month of January = 4.185 t and an annual period carbon dioxide supply amount = 40.77 t are found. The use rate calculation unit 12 outputs and displays "40.8 t" in a section of "$CO_2$ supply amount" on the output screen illustrated in FIG. 2 (S607).

The use rate calculation unit 12 calculates that the use rate is 10.10/40.77 = 24.8% in a case of a comparison by the year, and calculates that it is 0.652/4.185 = 15.6% in a case of a comparison with the one for a month of January, for example.

In an example of the output screen illustrated in FIG. 2, "$CO_2$ absorption amount: 10.1 t" is displayed as the annual carbon dioxide absorption amount, and "$CO_2$ supply amount: 40.8 t" is displayed as the annual carbon dioxide supply amount, and the use rate calculation unit 12 further outputs and displays an annual use rate as "25%" in a section of "$CO_2$ use rate".

According to the carbon dioxide dynamics simulation apparatus of this embodiment, the absorption amount and the use rate of carbon dioxide supplied to the greenhouse can be found in this manner. This makes it possible to clearly grasp the dynamics of carbon dioxide in the greenhouse. Further, the absorption amount and the use rate can be grasped, so that excessive supply of carbon dioxide not corresponding to the use rate can be suppressed, which, even when the device which supplies carbon dioxide by the combustion of the fossil fuel is used, is useful in making the number of operations and an output thereof proper. The result leads to suppression of emission of greenhouse effect gas more than necessary due to operating the device which supplies carbon dioxide.

Next, a carbon dioxide dynamics simulation apparatus 1 according to the other embodiment of the present invention which can simulate a contribution to a reduction in greenhouse effect gas in a case of using the exhaust gas by comparing the case of using the device which generates carbon dioxide by use of the fossil fuel with the case of using the exhaust gas from factories and the like, as the carbon dioxide supply source, in addition to finding the absorption amount and the use rate to grasp the dynamics of carbon dioxide will be described.

In this embodiment, as illustrated in FIG. 7, similarly to the above-described embodiment, it has the absorption amount calculation unit 11 and the use rate calculation unit 12, and a computer program which executes procedures to cause it to function as the fist greenhouse-effect-gas emission-amount calculation unit 13, the second greenhouse-effect-gas emission-amount calculation unit 14, the greenhouse-effect-gas reduction-amount calculation unit 15, and the cost calculation unit 16 is stored in the storage part 1b.

The first greenhouse-effect-gas emission-amount calculation unit 13 has a function of finding a first greenhouse effect gas emission amount in the case of providing the supply amount of carbon dioxide supplied into the greenhouse using the device which generates carbon dioxide by the combustion of the fossil fuel, and the second greenhouse-effect-gas emission-amount calculation unit 14 has a function of calculating a second greenhouse effect gas emission amount in the case of providing the supply amount using carbon dioxide separated and recovered from the exhaust gas emitted from the exhaust gas generation source. The greenhouse-effect-gas reduction-amount calculation unit 15 calculates a difference between the first greenhouse effect gas emission amount and the second greenhouse effect gas emission amount as a greenhouse effect gas reduction amount in the case of using the exhaust gas supplied from the exhaust gas generation source.

The first greenhouse effect gas emission amount corresponds to an amount excluding the absorption amount based on the plant in the supply amount into the greenhouse. The second greenhouse effect gas emission amount uses the exhaust gas, and thus an emission amount to the outdoors associated with ventilation is not an additional emission amount of carbon dioxide. As a result, the greenhouse effect gas emission amount decreases by the absorption caused by the plant, and the second greenhouse-effect-gas emission-amount calculation unit 14 calculates an amount corresponding to the absorption amount found by the absorption amount calculation unit 11 with a negative number, as the second greenhouse effect gas emission amount in a case of providing the entire amount in the supply amount into the greenhouse with the carbon dioxide separated and recovered from the exhaust gas. Thus, the greenhouse-effect-gas reduction-amount calculation unit 15 outputs, as a reduction amount, a value obtained by adding a value of an absolute value of the second greenhouse effect gas emission amount to a value of the first greenhouse effect gas emission amount. As a result, in the case of providing the entire amount of the carbon dioxide supply amount with the exhaust gas, the supply amount of carbon dioxide is the difference between the two, that is, the reduction amount by comparison with the device which uses the fossil fuel.

When the first greenhouse-effect-gas emission-amount calculation unit 13, the second greenhouse-effect-gas emission-amount calculation unit 14, and the greenhouse-effect-gas reduction-amount calculation unit 15 find the first greenhouse effect gas emission amount, the second greenhouse effect gas emission amount, and the greenhouse effect gas reduction amount, their respective values are output and displayed on the output screen illustrated in FIG. 2.

The cost calculation unit 16 calculates a cost required for the supply of carbon dioxide from the carbon dioxide supply amount. By comparing a carbon dioxide supply cost in the case of using the fossil fuel with a carbon dioxide supply cost in the case of using the exhaust gas, a difference between the two is found as a reduction cost of the carbon dioxide supply cost.

The carbon dioxide supply cost in the case of using the device which combusts the fossil fuel is found by a unit price of the fuel, and, a heating value and a carbon emission factor of the fuel.

A mass unit price of carbon dioxide in the case of using the exhaust gas can be determined by an agreement or the like among the parties in an enterprise of the exhaust gas generation source, a contractor of the greenhouse, administration, and the like. Further, it is also possible to refer to market prices of transactions of the carbon dioxide emission amount. Anyway, the carbon dioxide cost is set to be considerably more inexpensive than that in the case of using the fossil fuel.

Next, an example of simulating a reduction situation of the greenhouse effect gas by using the carbon dioxide dynamics simulation apparatus 1 of this embodiment will be described.

[Calculation of greenhouse effect gas reduction amount]

A difference between the greenhouse effect gas emission amount in the case of using the device which generates carbon dioxide by the combustion of the fossil fuel and the greenhouse effect gas emission amount in the case of using carbon dioxide from the exhaust gas, as the carbon dioxide supply source, is calculated as the greenhouse effect gas reduction amount.

(The case of using the device which generates carbon dioxide by use of the fossil fuel)

In the first greenhouse-effect-gas emission-amount calculation unit 13, it is found by the following formula.

The first greenhouse effect gas emission amount = carbon dioxide supply amount - carbon dioxide absorption amount

In comparison by the year using the above-described example, the first greenhouse effect gas emission amount is found as

$$40.77\ t - 10.10\ t = 30.67\ t.$$

(The case of providing the entire amount of the supply amount of carbon dioxide with the exhaust gas)

In the second greenhouse-effect-gas emission-amount calculation unit 14, it is found by the following formula.

The second greenhouse gas emission amount = -carbon dioxide absorption amount

In comparison by the year using the above-described example,
the second greenhouse effect gas emission amount = -10.10 t is found.

(Greenhouse effect gas reduction amount)

In the greenhouse-effect-gas reduction-amount calculation unit 15, it is found by the following formula.

The greenhouse effect gas reduction amount = the first greenhouse effect gas emission amount - the second greenhouse effect gas emission amount

In the above-described example, the greenhouse effect gas reduction amount is found as

$$30.67\ t - (-10.10\ t) = 40.77\ t.$$

Since this example is premised on providing the entire amount of the supply amount of carbon dioxide with the exhaust gas, the greenhouse effect gas reduction amount which is the difference of the first greenhouse effect gas emission amount - the second greenhouse effect gas emission amount is equal to the supply amount of carbon dioxide, but for example, in a case of failing to obtain the supply of a sufficient exhaust gas from the factory or the like depending on times, the carbon dioxide generated by combusting the fossil fuel compensates for the shortage. In this case, for example, when it is assumed that the fossil fuel is used for a month in a year of the cultivation period and the exhaust gas is used for the other months, a period greenhouse effect gas reduction amount is calculated by the "carbon dioxide supply amount - carbon dioxide absorption amount" similarly to the first greenhouse-effect-gas emission-amount calculation unit 13 regarding the one for a month, and a negative number of a value corresponding to the carbon dioxide absorption amount is calculated as a period greenhouse effect gas emission amount regarding the other months, resulting in that a value obtained by integrating both is found as the second greenhouse effect gas emission amount.

For example, when the annual carbon dioxide supply amount is set to 40.77 t the same as the above, and the annual carbon dioxide absorption amount is set to 10.10 t the same as the above, the period greenhouse effect gas emission amount for a month when carbon dioxide is supplied using the fossil fuel is "3.5 - 2.1 = 1.4 t" assuming that a period carbon dioxide supply amount for a month is 3.5 t and a period carbon dioxide absorption amount for a month is 2.1 t. The period greenhouse effect gas emission amount (period carbon dioxide absorption amount) for the other eleven months when the exhaust gas is used is "10.10 - 2.1 = -8 t". Consequently, "-6.6 t" obtained by adding both together is the second greenhouse effect gas emission amount, and the greenhouse effect gas reduction amount is "30.67 - (-6.6) = 37.27 t".

Thus, as illustrated in FIG. 2, as input information, it is preferable that a rate of exhaust gas use in the carbon dioxide supply amount can be input as a recycling rate.

In this case, the absorption amount calculation unit 11 integrates the daily carbon dioxide absorption amounts excluding a period corresponding to this recycling rate, and the second greenhouse-effect-gas emission-amount calculation unit 14 can find the second greenhouse effect gas emission amount in accordance with the recycling rate by adding the period when the fossil fuel is used and the supply is performed and the period when the exhaust gas is used and supplied together, as described above.

On the output screen illustrated in FIG. 2, in a section displayed as "$CO_2$ absorption amount", by the first greenhouse-effect-gas emission-amount calculation unit 13, the second greenhouse-effect-gas emission-amount calculation unit 14, and the greenhouse-effect-gas reduction-amount calculation unit 15, "30.7 t" is output as an annual greenhouse effect gas emission amount in the case of using the device which generates carbon dioxide by use of the fossil fuel (the absence of resource recycling), and "-10.1 t" which is a negative number of a value corresponding to the annual carbon dioxide absorption amount is output as an annual greenhouse effect gas emission amount in the case of using the exhaust gas at a recycling rate of 100% (the presence of resource recycling), and "40.8 t" is output and displayed as the greenhouse effect gas reduction amount, respectively.

[Calculation of reduction cost of carbon dioxide supply cost]

The cost calculation unit 16 compares the carbon dioxide supply cost in the case of providing the above-described carbon dioxide supply amount by using the device which generates carbon dioxide by the combustion of the fossil fuel with the carbon dioxide supply cost in the case of providing it using the exhaust gas.

(The case of using the device which generates carbon dioxide by use of the fossil fuel)

For example, in a case of a device using an LPG as the fuel, when a fuel unit price of the LPG is set to ¥256/kg, according to a heating value of 50.08 MJ/kg and a carbon emission factor; 0.01637 kgC/MJ, a mass unit price of carbon dioxide is

$$256/(0.01637 \times 50.08 \times 44/12) = ¥85.$$

When the supply amount of carbon dioxide is set to the period carbon dioxide supply amount for a month of January = 4.185 t and the annual period carbon dioxide supply amount = 40.77 t according to the above-described example, a calculation is made as follows.

$$\text{A monthly carbon dioxide supply cost of January} = ¥85 \times 4185\ \text{kg} = ¥355{,}725$$

$$\text{An annual carbon dioxide supply cost} = ¥85 \times 40770\ \text{kg} = ¥3{,}465{,}450$$

(The case of providing the entire amount of the supply amount of carbon dioxide with the exhaust gas)

Assuming that the mass unit price of carbon dioxide is ¥38 from a transaction price of the exhaust gas, a calculation is made similarly to the above.

$$\text{The monthly carbon dioxide supply cost of January} = ¥38 \times 4185\ \text{kg} = ¥159{,}030$$

$$\text{The annual carbon dioxide supply cost} = ¥38 \times 40770\ \text{kg} = ¥1{,}549{,}260$$

(Reduction cost of carbon dioxide supply cost) A monthly carbon dioxide reduction cost of January = ¥355,725 - ¥159,030 = ¥196,695 An annual carbon dioxide reduction cost = ¥3,465,450 - ¥1,549,260 = ¥1,916,190

Note that in a case of setting the recycling rate, the supply cost of carbon dioxide and the reduction cost of the carbon dioxide supply cost are calculated according to the recycling rate, similarly to the case of finding the above-described greenhouse effect gas emission amounts and greenhouse effect gas reduction amount.

On the output screen illustrated in FIG. 2, in a section displayed as "$CO_2$ cost", by the cost calculation unit 16, "¥3,465,450" is output as an annual carbon dioxide supply cost in the case of using the device which generates carbon dioxide by the use of the fossil fuel (the absence of resource recycling), "¥1,549,260" is output as an annual carbon dioxide supply cost in the case of using the exhaust gas at a recycling rate of 100% (the presence of resource recycling), and "¥1,916,190" is output and displayed as the reduction cost.

According to the above, the carbon dioxide dynamics simulation apparatus 1 of this embodiment can quantitatively clearly indicate, in the case of providing the carbon dioxide supply amount with the exhaust gas, how much the amount can be reduced and how much the supply cost of carbon dioxide can be reduced further than in the case of using the device which supplies carbon dioxide using the fossil fuel.

Therefore, in this embodiment, a merit in constructing the greenhouse which recycles the exhaust gas can be clarified, which causes an incentive to promote the construction of the greenhouse capable of contributing to the reduction in greenhouse effect gas. Further, the factories which emit the exhaust gas, and the like also allow the greenhouse effect gas to be reduced, which can lead to activation of the transaction of the carbon dioxide emission amount. That is, "¥1,549,260" which is an example of the annual carbon dioxide supply cost in the case of the above-described "presence of resource recycling" is new proceeds based on the exhaust gas which is only emitted into the air conventionally when seen from the factory or the like which is an exhaust gas providing side. Consequently, in the output screen in FIG. 2, an item displaying this amount of money as gross proceeds in the exhaust gas providing side is preferably provided. Allowing such a numerical value to be grasped before the construction of the greenhouse results in an incentive for the exhaust gas providing side to be in cooperation with the greenhouse.

Explanation of Reference Signs

1 carbon dioxide dynamics simulation apparatus
11 absorption amount calculation unit
111 house information acquisition unit
112 plant information acquisition unit
113 weather information acquisition unit
12 use rate calculation unit
13 first greenhouse-effect-gas emission-amount calculation unit
14 second greenhouse-effect-gas emission-amount calculation unit
15 greenhouse-effect-gas reduction-amount calculation unit
16 cost calculation unit

## Claims

**1.** A carbon dioxide dynamics simulation apparatus which simulates dynamics of carbon dioxide in a greenhouse, the carbon dioxide dynamics simulation apparatus comprising:

an absorption amount calculation unit which finds an amount of net photosynthesis of a plant cultivated in the greenhouse as an absorption amount of carbon dioxide based on the plant; and
a use rate calculation unit which finds a rate of the absorption amount to a supply amount of carbon dioxide supplied into the greenhouse from a carbon dioxide supply source during a predetermined period as a use rate of the carbon dioxide supplied to the greenhouse.

**2.** The carbon dioxide dynamics simulation apparatus according to claim 1, wherein the absorption amount calculation unit finds the amount of net photosynthesis using a global solar irradiance amount, a light transmittance of the greenhouse, a light interception rate of a plant, and an indoor carbon dioxide concentration in the greenhouse.

**3.** The carbon dioxide dynamics simulation apparatus according to claim 2, wherein:

the indoor carbon dioxide concentration is calculated based on an indoor-outdoor ventilation rate of air in the greenhouse; and
the ventilation rate is calculated using a difference between an outdoor specific enthalpy and an indoor specific enthalpy of the greenhouse.

**4.** The carbon dioxide dynamics simulation apparatus according to claim 2, wherein the absorption amount calculation unit comprises:

a house structure information acquisition unit which acquires structure information of the greenhouse including the light transmittance;
a plant information acquisition unit which acquires information of the plant targeted for cultivation including the light interception rate; and
a weather information acquisition unit which acquires weather information in an installation region of the greenhouse.

**5.** The carbon dioxide dynamics simulation apparatus according to claim 1, comprising:

a first greenhouse-effect-gas emission-amount calculation unit which finds a first greenhouse effect gas emission amount in a case of providing a supply amount of carbon dioxide supplied into the greenhouse using a device which generates carbon dioxide by combustion of a fossil fuel as the carbon dioxide supply source;
a second greenhouse-effect-gas emission-amount calculation unit which finds a second greenhouse effect gas emission amount in a case of providing a supply amount of carbon dioxide supplied into the greenhouse with carbon dioxide recovered from an exhaust gas emitted from an exhaust gas generation source as the carbon dioxide supply source; and
a greenhouse-effect-gas reduction-amount calculation unit which calculates a difference between the first greenhouse effect gas emission amount and the second greenhouse effect gas emission amount as a greenhouse effect gas reduction amount in the case of using the exhaust gas.

**6.** The carbon dioxide dynamics simulation apparatus according to claim 5, wherein the first greenhouse-effect-gas emission-amount calculation unit finds a difference between a supply amount of the carbon dioxide and an absorption amount of the carbon dioxide as the first greenhouse effect gas emission amount.

**7.** The carbon dioxide dynamics simulation apparatus according to claim 5, wherein the second greenhouse-effect-gas emission-amount calculation unit finds the second greenhouse effect gas emission amount by setting a value corresponding to an absorption amount of the carbon dioxide as a negative number.

**8.** The carbon dioxide dynamics simulation apparatus according to claim 5, comprising a cost calculation unit which finds a reduction cost of a carbon dioxide supply cost by comparing the case of supplying a supply amount of the carbon dioxide using the fossil fuel with the case of supplying a supply amount of the carbon dioxide using the exhaust gas.

**9.** A computer program which causes a computer to function as a carbon dioxide dynamics simulation apparatus in a greenhouse, the computer program causing the computer to execute

a procedure for finding an amount of net photosynthesis of a plant cultivated in the greenhouse as an absorption amount of carbon dioxide based on the plant, and
a procedure for finding a rate of the absorption amount to a supply amount of carbon dioxide supplied into the greenhouse from a carbon dioxide supply source during a predetermined period as a use rate of the carbon dioxide supplied to the greenhouse.

**10.** The computer program according to claim 9, wherein in the procedure for finding an absorption amount of the carbon dioxide, the amount of net photosynthesis is found using a global solar irradiance amount, a light transmittance of the greenhouse, a light interception rate of a plant, and an indoor carbon dioxide concentration in the greenhouse.

**11.** The computer program according to claim 10, wherein:

the indoor carbon dioxide concentration is calculated based on an indoor-outdoor ventilation rate of air in the greenhouse; and
the ventilation rate is calculated using a difference between an outdoor specific enthalpy and an indoor specific enthalpy of the greenhouse.

**12.** The computer program according to claim 10, causing the computer to execute,

in the procedure for finding an absorption amount of the carbon dioxide,
a procedure for acquiring structure information of the greenhouse including the light transmittance,
a procedure for acquiring information of the plant targeted for cultivation including the light interception rate, and
a procedure for acquiring weather information in an installation region of the greenhouse.

**13.** The computer program according to claim 9, causing the computer to further execute

a procedure for finding a first greenhouse effect gas emission amount in a case of providing a supply amount of carbon dioxide supplied into the greenhouse using a device which generates carbon dioxide by combustion of a fossil fuel as the carbon dioxide supply source,
a procedure for finding a second greenhouse effect gas emission amount in a case of providing a supply amount of carbon dioxide supplied into the greenhouse with carbon dioxide recovered from an exhaust gas emitted from an exhaust gas generation source as the carbon dioxide supply source, and
a procedure for calculating a difference between the first greenhouse effect gas emission amount and the second greenhouse effect gas emission amount as a greenhouse effect gas reduction amount in the case of using the exhaust gas.

**14.** The computer program according to claim 13, causing the computer to further execute a procedure for finding a reduction cost of a carbon dioxide supply cost by comparing the case of supplying a supply amount of the carbon dioxide using the fossil fuel with the case of supplying a supply amount of the carbon dioxide using the exhaust gas.

**15.** A computer-readable recording medium in which the computer program according to any one of claims 9 to 14 is recorded.

FIG. 1

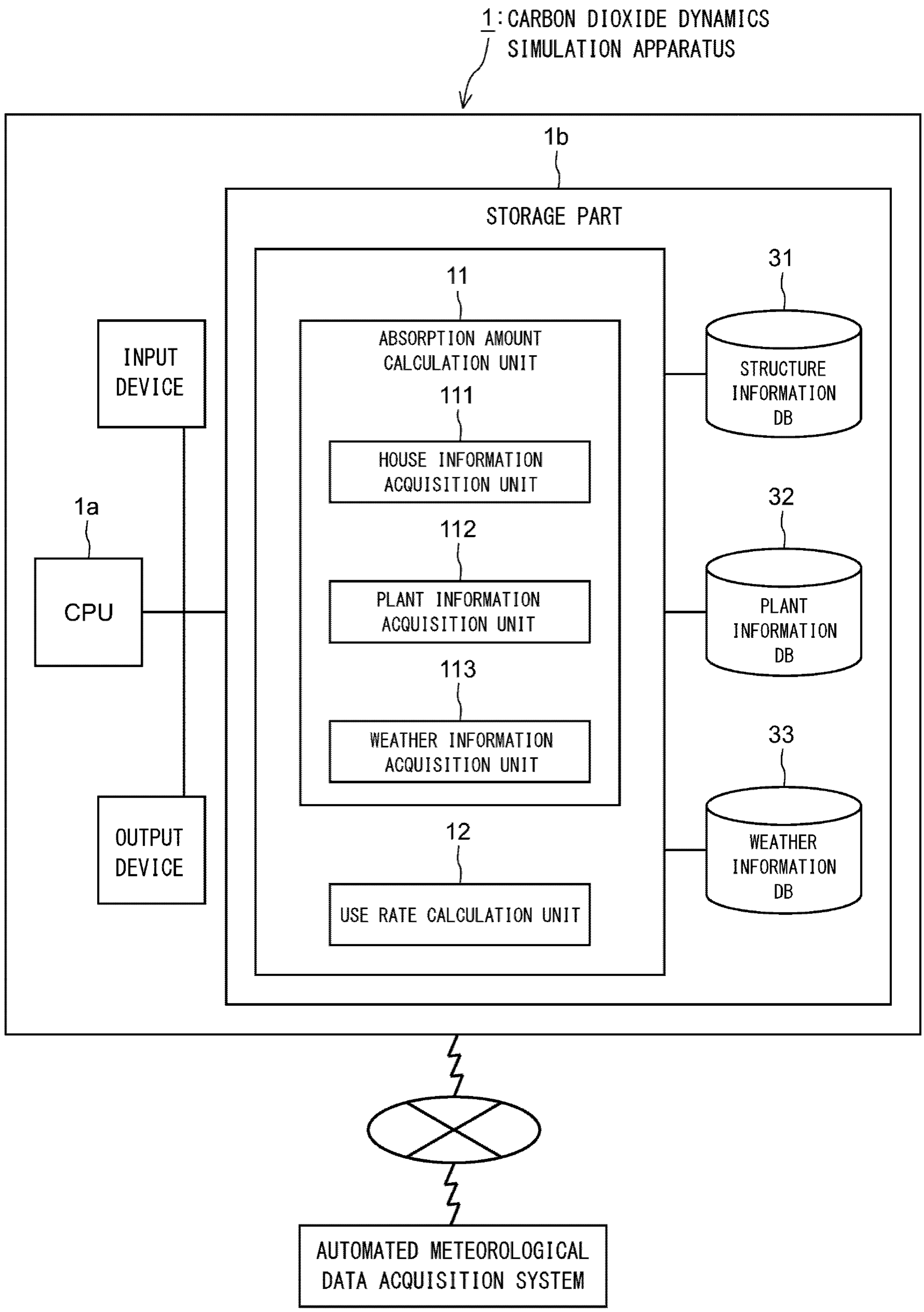
1: CARBON DIOXIDE DYNAMICS SIMULATION APPARATUS
1b
STORAGE PART
11
ABSORPTION AMOUNT CALCULATION UNIT
111
HOUSE INFORMATION ACQUISITION UNIT
112
PLANT INFORMATION ACQUISITION UNIT
113
WEATHER INFORMATION ACQUISITION UNIT
12
USE RATE CALCULATION UNIT
31
STRUCTURE INFORMATION DB
32
PLANT INFORMATION DB
33
WEATHER INFORMATION DB
INPUT DEVICE
1a
CPU
OUTPUT DEVICE
AUTOMATED METEOROLOGICAL DATA ACQUISITION SYSTEM

FIG. 2

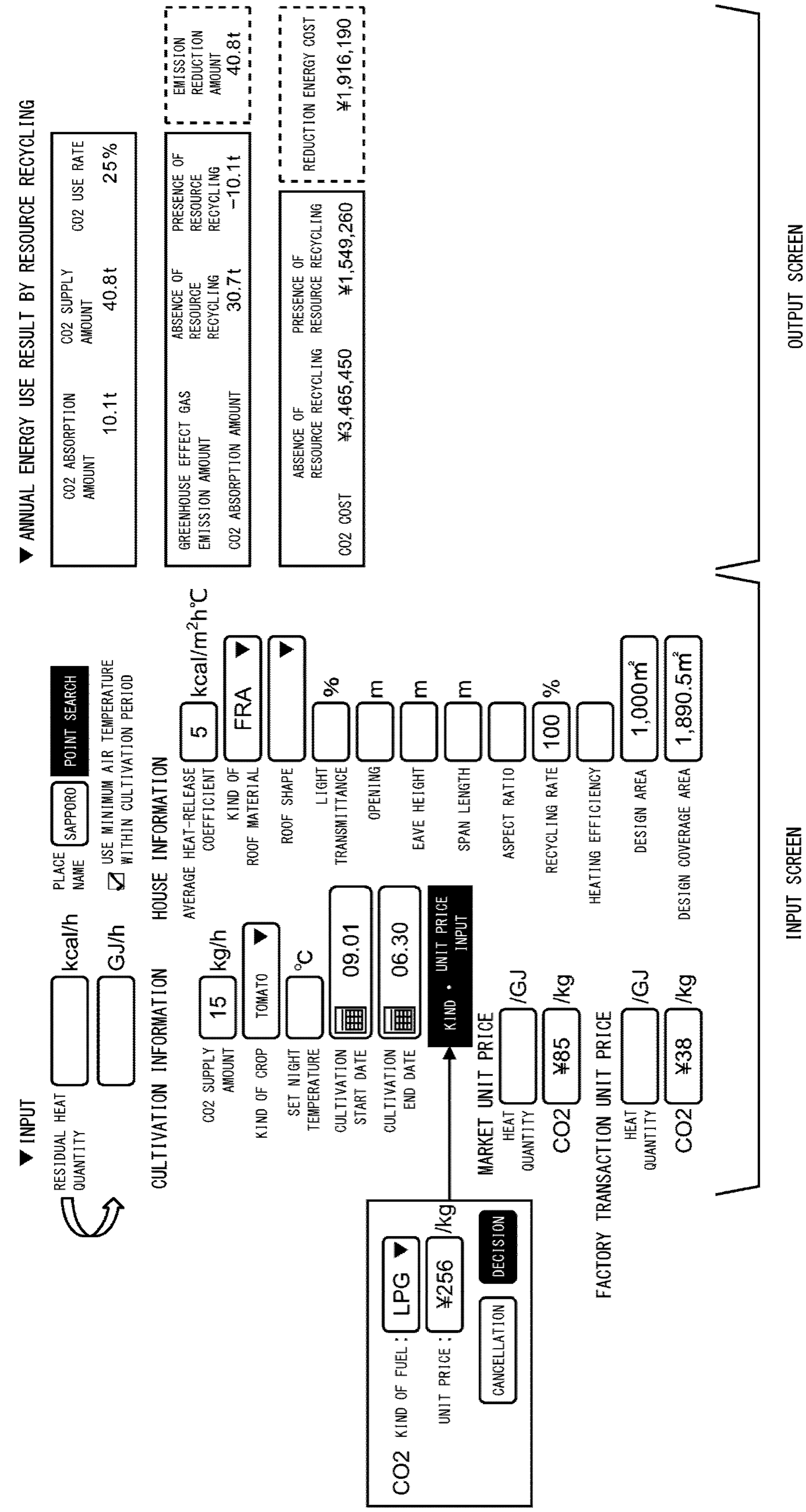

▼ INPUT
RESIDUAL HEAT QUANTITY
kcal/h
GJ/h
PLACE NAME
SAPPORO
POINT SEARCH
USE MINIMUM AIR TEMPERATURE WITHIN CULTIVATION PERIOD
CULTIVATION INFORMATION
CO2 SUPPLY AMOUNT
15
kg/h
KIND OF CROP
TOMATO
SET NIGHT TEMPERATURE
°C
CULTIVATION START DATE
09.01
CULTIVATION END DATE
06.30
KIND · UNIT PRICE INPUT
MARKET UNIT PRICE
HEAT QUANTITY
/GJ
CO2
¥85
/kg
FACTORY TRANSACTION UNIT PRICE
HEAT QUANTITY
/GJ
CO2
¥38
/kg
CO2 KIND OF FUEL :
LPG
UNIT PRICE :
¥256
/kg
CANCELLATION
DECISION
HOUSE INFORMATION
AVERAGE HEAT-RELEASE COEFFICIENT
5
kcal/m²h°C
KIND OF ROOF MATERIAL
FRA
ROOF SHAPE
LIGHT TRANSMITTANCE
%
OPENING
m
EAVE HEIGHT
m
SPAN LENGTH
m
ASPECT RATIO
RECYCLING RATE
100
%
HEATING EFFICIENCY
DESIGN AREA
1,000m²
DESIGN COVERAGE AREA
1,890.5m²
INPUT SCREEN
▼ ANNUAL ENERGY USE RESULT BY RESOURCE RECYCLING
CO2 ABSORPTION AMOUNT
10.1t
CO2 SUPPLY AMOUNT
40.8t
CO2 USE RATE
25%
GREENHOUSE EFFECT GAS EMISSION AMOUNT
CO2 ABSORPTION AMOUNT
ABSENCE OF RESOURCE RECYCLING
30.7t
PRESENCE OF RESOURCE RECYCLING
-10.1t
EMISSION REDUCTION AMOUNT
40.8t
CO2 COST
ABSENCE OF RESOURCE RECYCLING
¥3,465,450
PRESENCE OF RESOURCE RECYCLING
¥1,549,260
REDUCTION ENERGY COST
¥1,916,190
OUTPUT SCREEN

FIG. 3

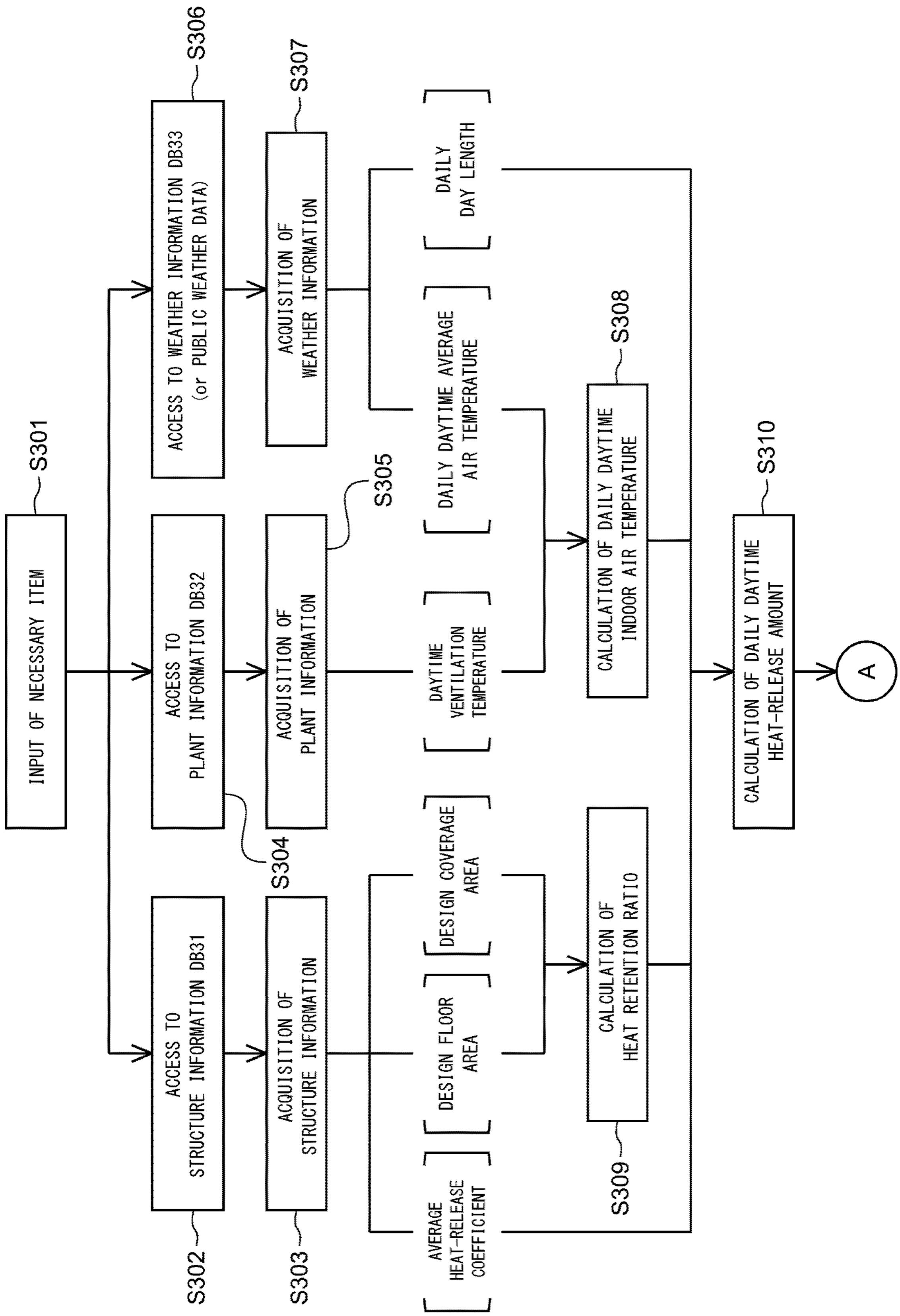
INPUT OF NECESSARY ITEM
S301
ACCESS TO STRUCTURE INFORMATION DB31
S302
ACCESS TO PLANT INFORMATION DB32
S304
ACCESS TO WEATHER INFORMATION DB33 (or PUBLIC WEATHER DATA)
S306
ACQUISITION OF STRUCTURE INFORMATION
S303
ACQUISITION OF PLANT INFORMATION
S305
ACQUISITION OF WEATHER INFORMATION
S307
AVERAGE HEAT-RELEASE COEFFICIENT
DESIGN FLOOR AREA
DESIGN COVERAGE AREA
DAYTIME VENTILATION TEMPERATURE
DAILY DAYTIME AVERAGE AIR TEMPERATURE
DAILY DAY LENGTH
CALCULATION OF HEAT RETENTION RATIO
S309
CALCULATION OF DAILY DAYTIME INDOOR AIR TEMPERATURE
S308
CALCULATION OF DAILY DAYTIME HEAT-RELEASE AMOUNT
S310
A

FIG. 4

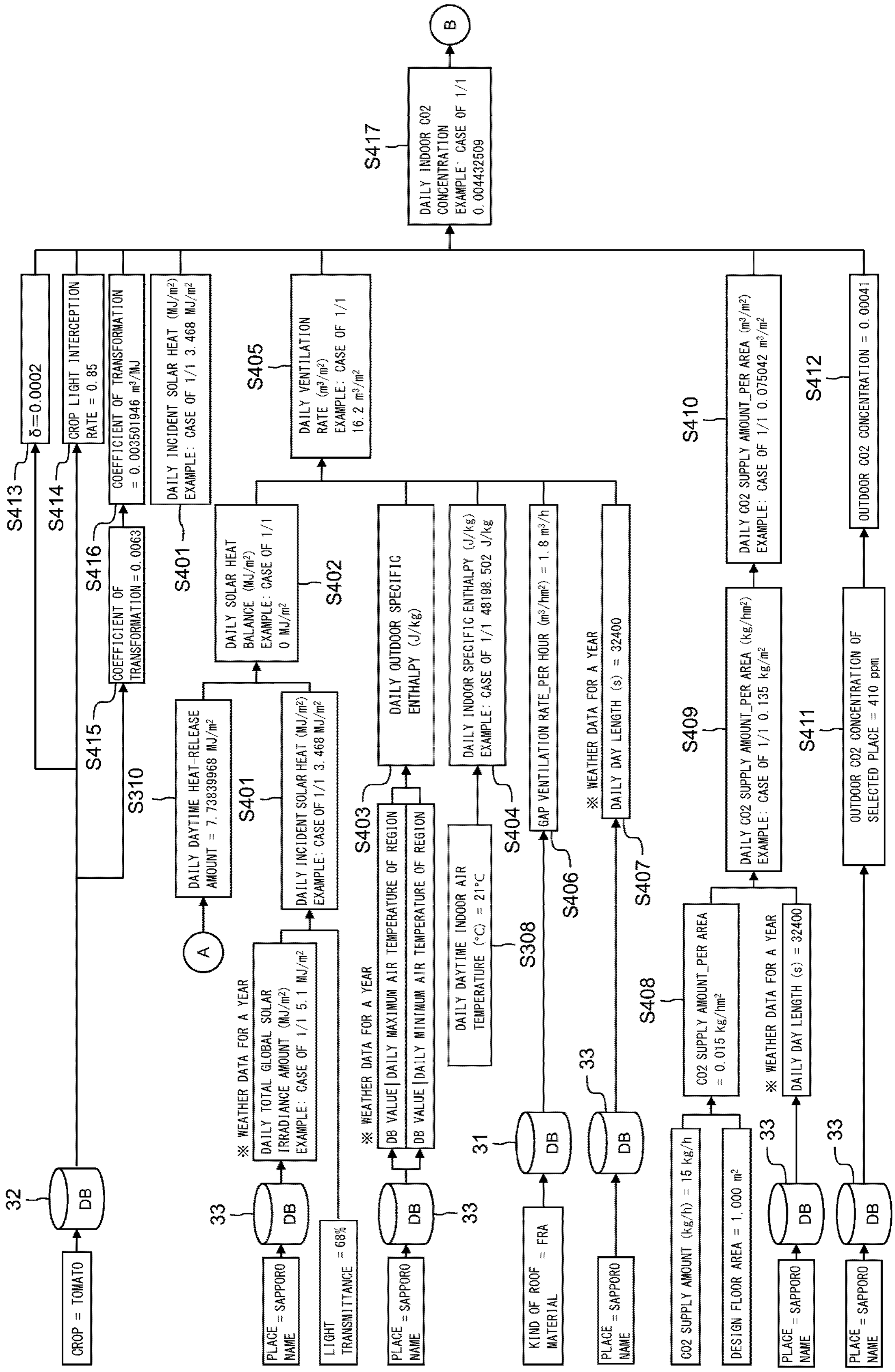
B
S417
DAILY INDOOR CO2 CONCENTRATION EXAMPLE: CASE OF 1/1 0.004432509
S413
δ=0.0002
S414
CROP LIGHT INTERCEPTION RATE = 0.85
COEFFICIENT OF TRANSFORMATION = 0.003501946 m³/MJ
S416
COEFFICIENT OF TRANSFORMATION = 0.0063
S415
S401
DAILY INCIDENT SOLAR HEAT (MJ/m²) EXAMPLE: CASE OF 1/1 3.468 MJ/m²
S405
DAILY VENTILATION RATE (m³/m²) EXAMPLE: CASE OF 1/1 16.2 m³/m²
S402
DAILY SOLAR HEAT BALANCE (MJ/m²) EXAMPLE: CASE OF 1/1 0 MJ/m²
S310
DAILY DAYTIME HEAT-RELEASE AMOUNT = 7.73839968 MJ/m²
S401
DAILY INCIDENT SOLAR HEAT (MJ/m²) EXAMPLE: CASE OF 1/1 3.468 MJ/m²
A
※ WEATHER DATA FOR A YEAR
DAILY TOTAL GLOBAL SOLAR IRRADIANCE AMOUNT (MJ/m²) EXAMPLE: CASE OF 1/1 5.1 MJ/m²
S403
DAILY OUTDOOR SPECIFIC ENTHALPY (J/kg)
※ WEATHER DATA FOR A YEAR
DB VALUE DAILY MAXIMUM AIR TEMPERATURE OF REGION
DB VALUE DAILY MINIMUM AIR TEMPERATURE OF REGION
S404
DAILY INDOOR SPECIFIC ENTHALPY (J/kg) EXAMPLE: CASE OF 1/1 48198.502 J/kg
S308
DAILY DAYTIME INDOOR AIR TEMPERATURE (°C) = 21°C
S406
GAP VENTILATION RATE_PER HOUR (m³/hm²) = 1.8 m³/h
S407
※ WEATHER DATA FOR A YEAR
DAILY DAY LENGTH (s) = 32400
S410
DAILY CO2 SUPPLY AMOUNT_PER AREA (m³/m²) EXAMPLE: CASE OF 1/1 0.075042 m³/m²
S409
DAILY CO2 SUPPLY AMOUNT_PER AREA (kg/hm²) EXAMPLE: CASE OF 1/1 0.135 kg/m²
S408
CO2 SUPPLY AMOUNT_PER AREA = 0.015 kg/hm²
※ WEATHER DATA FOR A YEAR
DAILY DAY LENGTH (s) = 32400
S412
OUTDOOR CO2 CONCENTRATION = 0.00041
S411
OUTDOOR CO2 CONCENTRATION OF SELECTED PLACE = 410 ppm
32
DB
CROP = TOMATO
33
DB
PLACE NAME = SAPPORO
LIGHT TRANSMITTANCE = 68%
33
DB
PLACE NAME = SAPPORO
31
DB
KIND OF ROOF MATERIAL = FRA
33
DB
PLACE NAME = SAPPORO
CO2 SUPPLY AMOUNT (kg/h) = 15 kg/h
DESIGN FLOOR AREA = 1,000 m²
33
DB
PLACE NAME = SAPPORO
33
DB
PLACE NAME = SAPPORO

FIG. 5

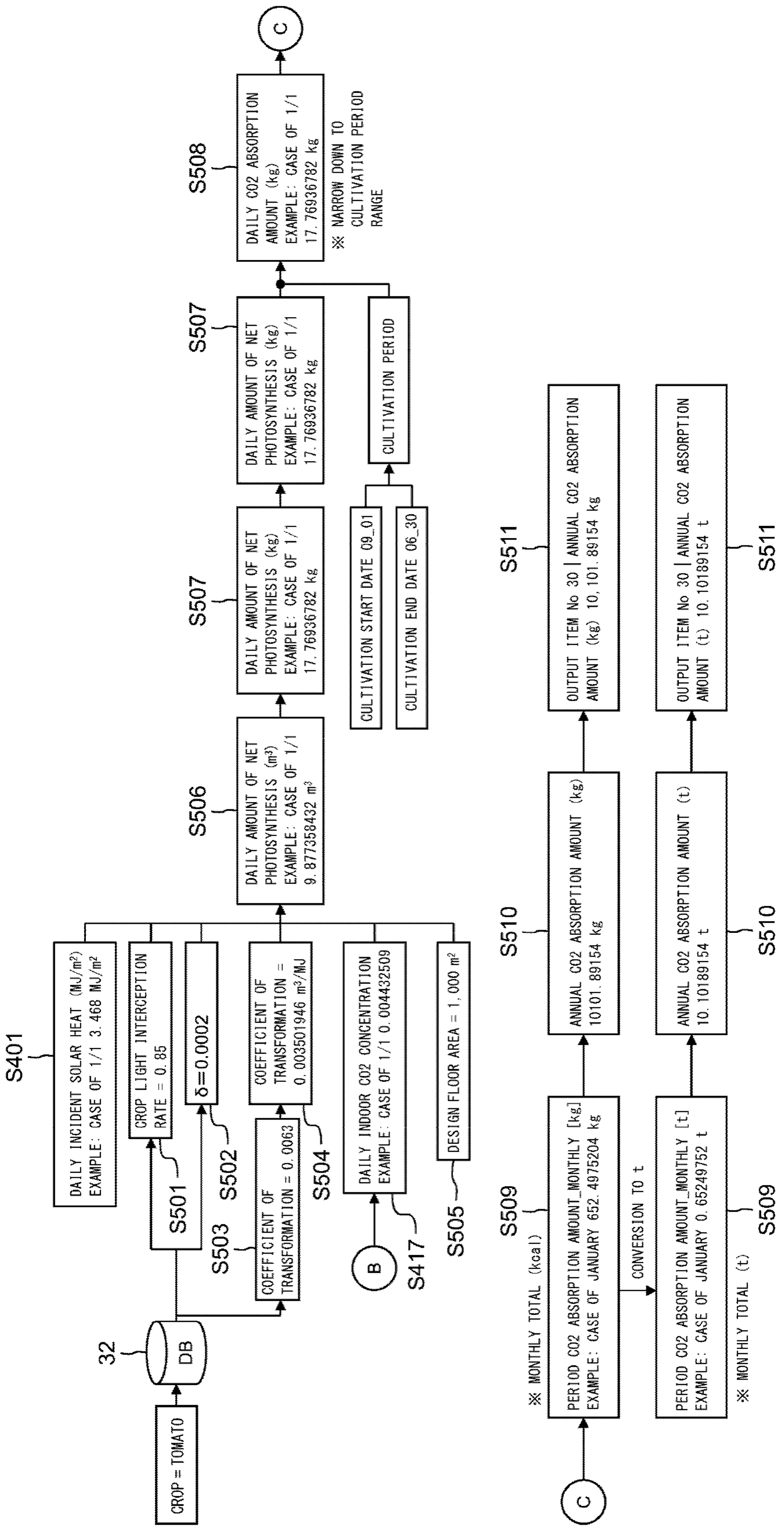
CROP = TOMATO
32
DB
S401
DAILY INCIDENT SOLAR HEAT (MJ/m²)
EXAMPLE: CASE OF 1/1 3.468 MJ/m²
S501
CROP LIGHT INTERCEPTION RATE = 0.85
S502
δ=0.0002
S503
COEFFICIENT OF TRANSFORMATION = 0.0063
S504
COEFFICIENT OF TRANSFORMATION = 0.003501946 m³/MJ
B
S417
DAILY INDOOR CO2 CONCENTRATION
EXAMPLE: CASE OF 1/1 0.004432509
S505
DESIGN FLOOR AREA = 1,000 m²
S506
DAILY AMOUNT OF NET PHOTOSYNTHESIS (m³)
EXAMPLE: CASE OF 1/1
9.877358432 m³
S507
DAILY AMOUNT OF NET PHOTOSYNTHESIS (kg)
EXAMPLE: CASE OF 1/1
17.76936782 kg
S507
DAILY AMOUNT OF NET PHOTOSYNTHESIS (kg)
EXAMPLE: CASE OF 1/1
17.76936782 kg
CULTIVATION START DATE 09_01
CULTIVATION END DATE 06_30
CULTIVATION PERIOD
S508
DAILY CO2 ABSORPTION AMOUNT (kg)
EXAMPLE: CASE OF 1/1
17.76936782 kg
※ NARROW DOWN TO CULTIVATION PERIOD RANGE
C
C
S509
※ MONTHLY TOTAL (kcal)
PERIOD CO2 ABSORPTION AMOUNT_MONTHLY [kg]
EXAMPLE: CASE OF JANUARY 652.4975204 kg
CONVERSION TO t
S510
ANNUAL CO2 ABSORPTION AMOUNT (kg)
10101.89154 kg
S511
OUTPUT ITEM No 30 | ANNUAL CO2 ABSORPTION AMOUNT (kg) 10,101.89154 kg
S509
PERIOD CO2 ABSORPTION AMOUNT_MONTHLY [t]
EXAMPLE: CASE OF JANUARY 0.65249752 t
※ MONTHLY TOTAL (t)
S510
ANNUAL CO2 ABSORPTION AMOUNT (t)
10.10189154 t
S511
OUTPUT ITEM No 30 | ANNUAL CO2 ABSORPTION AMOUNT (t) 10.10189154 t

FIG. 6

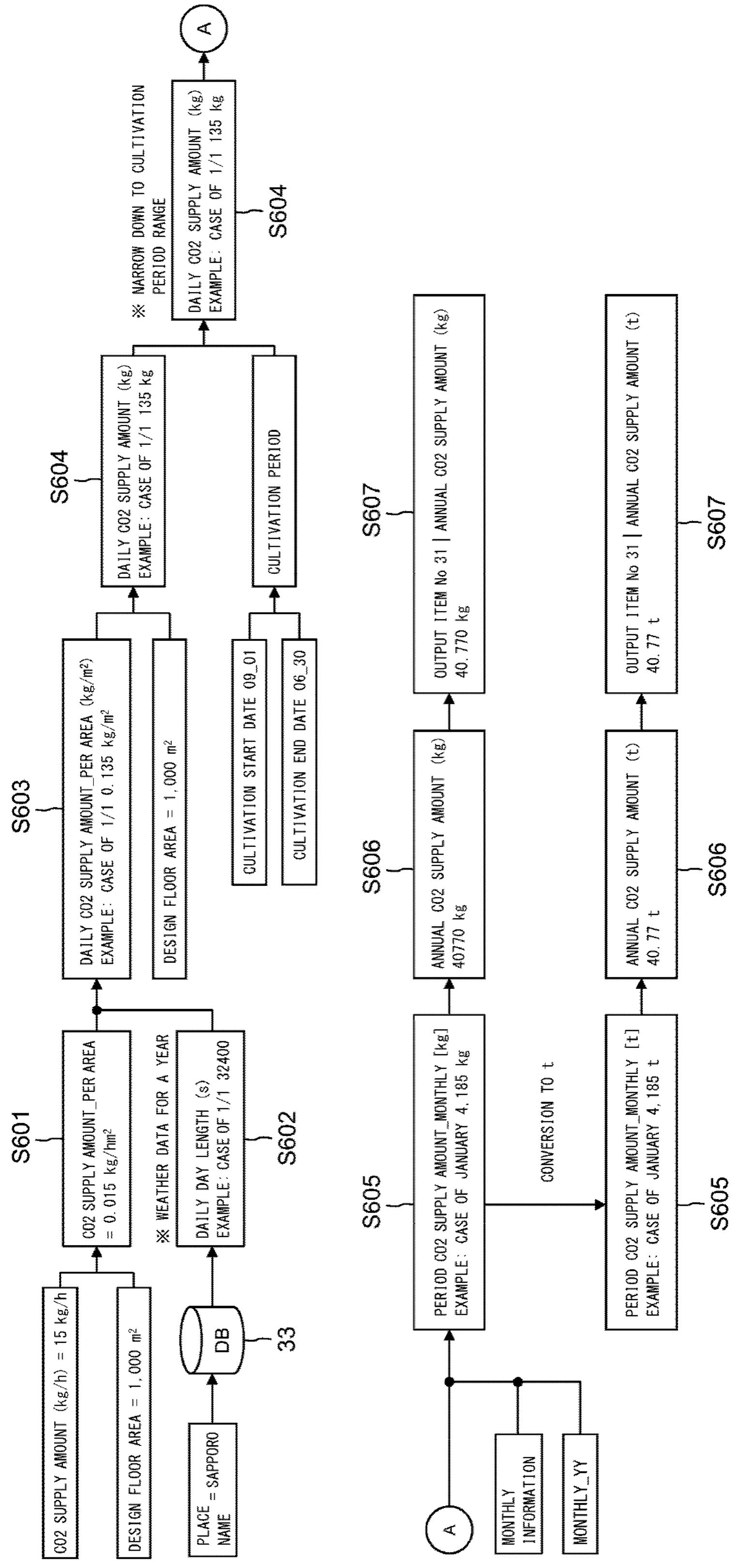

CO2 SUPPLY AMOUNT (kg/h) = 15 kg/h
DESIGN FLOOR AREA = 1,000 m²
PLACE NAME = SAPPORO
DB
33
S601
CO2 SUPPLY AMOUNT_PER AREA = 0.015 kg/hm²
※ WEATHER DATA FOR A YEAR
S602
DAILY DAY LENGTH (s) EXAMPLE: CASE OF 1/1 32400
S603
DAILY CO2 SUPPLY AMOUNT_PER AREA (kg/m²) EXAMPLE: CASE OF 1/1 0.135 kg/m²
DESIGN FLOOR AREA = 1,000 m²
CULTIVATION START DATE 09_01
CULTIVATION END DATE 06_30
S604
DAILY CO2 SUPPLY AMOUNT (kg) EXAMPLE: CASE OF 1/1 135 kg
CULTIVATION PERIOD
※ NARROW DOWN TO CULTIVATION PERIOD RANGE
S604
DAILY CO2 SUPPLY AMOUNT (kg) EXAMPLE: CASE OF 1/1 135 kg
A
A
MONTHLY INFORMATION
MONTHLY_YY
S605
PERIOD CO2 SUPPLY AMOUNT_MONTHLY [kg] EXAMPLE: CASE OF JANUARY 4,185 kg
CONVERSION TO t
S605
PERIOD CO2 SUPPLY AMOUNT_MONTHLY [t] EXAMPLE: CASE OF JANUARY 4,185 t
S606
ANNUAL CO2 SUPPLY AMOUNT (kg) 40770 kg
S606
ANNUAL CO2 SUPPLY AMOUNT (t) 40.77 t
S607
OUTPUT ITEM No 31 | ANNUAL CO2 SUPPLY AMOUNT (kg) 40.770 kg
S607
OUTPUT ITEM No 31 | ANNUAL CO2 SUPPLY AMOUNT (t) 40.77 t

FIG. 7

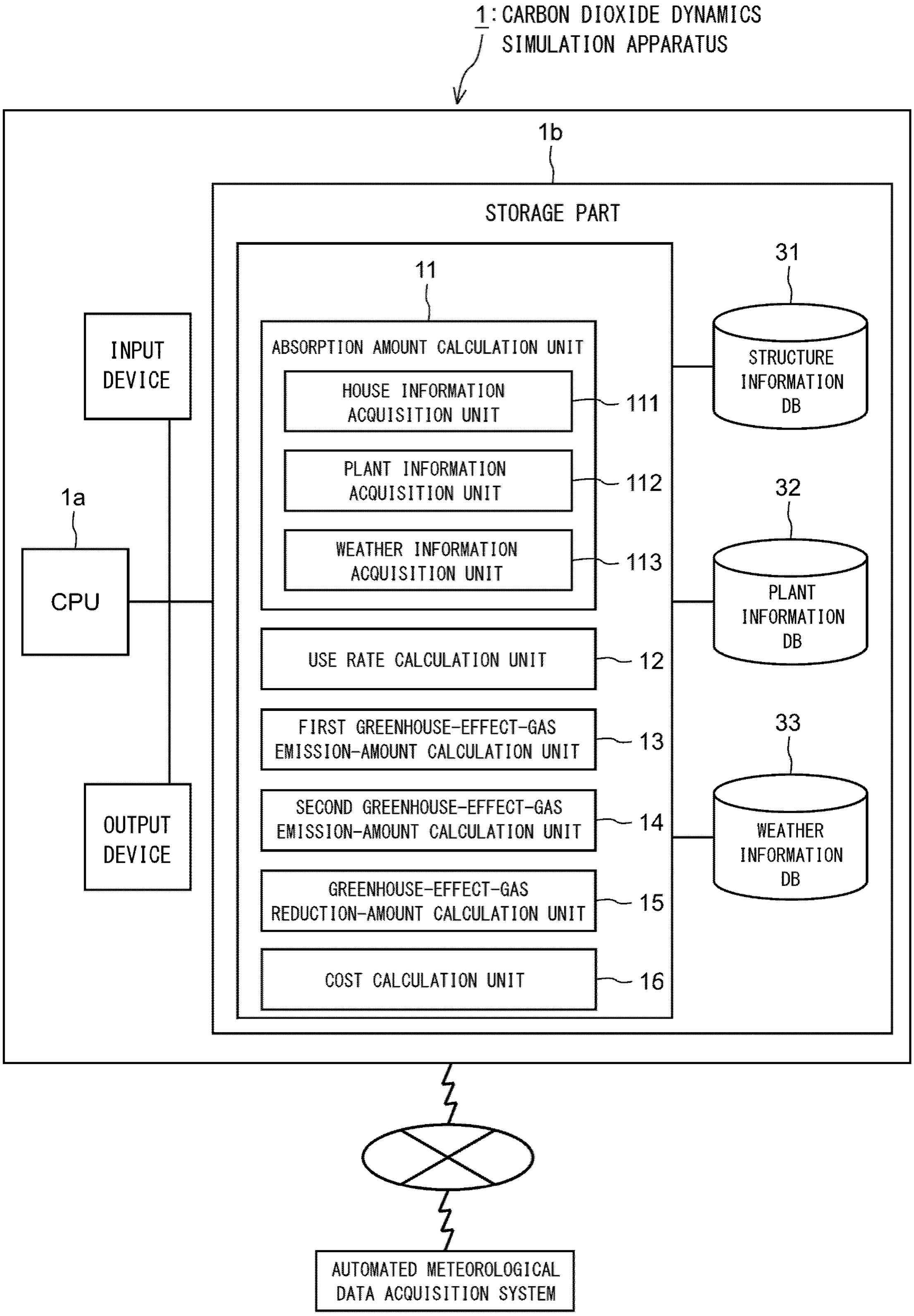
1: CARBON DIOXIDE DYNAMICS SIMULATION APPARATUS
1b
STORAGE PART
11
ABSORPTION AMOUNT CALCULATION UNIT
HOUSE INFORMATION ACQUISITION UNIT
111
PLANT INFORMATION ACQUISITION UNIT
112
WEATHER INFORMATION ACQUISITION UNIT
113
USE RATE CALCULATION UNIT
12
FIRST GREENHOUSE-EFFECT-GAS EMISSION-AMOUNT CALCULATION UNIT
13
SECOND GREENHOUSE-EFFECT-GAS EMISSION-AMOUNT CALCULATION UNIT
14
GREENHOUSE-EFFECT-GAS REDUCTION-AMOUNT CALCULATION UNIT
15
COST CALCULATION UNIT
16
31
STRUCTURE INFORMATION DB
32
PLANT INFORMATION DB
33
WEATHER INFORMATION DB
INPUT DEVICE
1a
CPU
OUTPUT DEVICE
AUTOMATED METEOROLOGICAL DATA ACQUISITION SYSTEM

**INTERNATIONAL SEARCH REPORT**

International application No. **PCT/JP2023/044184**

**A. CLASSIFICATION OF SUBJECT MATTER**

***G06Q 50/02***(2024.01)i
FI: G06Q50/02

According to International Patent Classification (IPC) or to both national classification and IPC

**B. FIELDS SEARCHED**

Minimum documentation searched (classification system followed by classification symbols)

G06Q50/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2019-44184 A (SANYO CHEMICAL INDUSTRIES, LTD.) 22 March 2019 (2019-03-22) entire text, all drawings | 1-15 |
| A | JP 8-172913 A (TOKYO GAS CO., LTD.) 09 July 1996 (1996-07-09) entire text, all drawings | 1-15 |
| A | WO 2018/021142 A1 (THE UNIVERSITY OF TOKYO) 01 February 2018 (2018-02-01) entire text, all drawings | 1-15 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 January 2024** | **06 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/044184**

| Patent document cited in search report | Publication date (day/month/year) | Patent family member(s) | Publication date (day/month/year) |
|---|---|---|---|
| JP 2019-44184 A | 22 March 2019 | (Family: none) | |
| JP 8-172913 A | 09 July 1996 | (Family: none) | |
| WO 2018/021142 A1 | 01 February 2018 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006212524 A
- JP 3236723 A
- JP 2021133314 A


### Non-patent literature cited in the description


- Tomato grows more and more rapidly with CO2! What is the latest technology of killing three birds with one stone?. *ENERGY FRONTLINE*, 13 April 2021, vol. 25, https://ene-fro.com/article/ef195_a1